(19) Europäisches Patentamt
European Patent Office
Office européen des brevets

(11) **EP 4 109 457 A1**

(12) **EUROPEAN PATENT APPLICATION**
published in accordance with Art. 153(4) EPC

(43) Date of publication:
**28.12.2022 Bulletin 2022/52**

(21) Application number: **20929446.1**

(22) Date of filing: **31.03.2020**

(51) International Patent Classification (IPC):
***G16H 10/40*** *(2018.01)*

(52) Cooperative Patent Classification (CPC):
**G16H 10/40**

(86) International application number:
**PCT/JP2020/014901**

(87) International publication number:
**WO 2021/199324 (07.10.2021 Gazette 2021/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Nippon Telegraph And Telephone Corporation**
**Chiyoda-ku**
**Tokyo 100-8116 (JP)**

(72) Inventors:
- **MOGAKI, Takefumi**
**Musashino-shi, Tokyo 180-8585 (JP)**
- **NAGATA, Kengo**
**Musashino-shi, Tokyo 180-8585 (JP)**
- **ITO, Hitoshi**
**Musashino-shi, Tokyo 180-8585 (JP)**

(74) Representative: **Brevalex**
**56, Boulevard de l'Embouchure**
**B.P. 27519**
**31075 Toulouse Cedex 2 (FR)**

# (54) CLINICAL EXAMINATION ITEM DETERMINATION DEVICE, HEALTHY BEHAVIOR ASSISTING DEVICE, CLINICAL EXAMINATION ITEM DETERMINATION METHOD, HEALTHY BEHAVIOR ASSISTING METHOD, AND COMPUTER PROGRAM

(57) An aspect of the present invention is a clinical examination item determination device that determines a clinical examination item for each user, the clinical examination item determination device including an individual examination item determination unit that estimates, based on genetic factors of the user and nongenetic factors based on behaviors or habits of the user, a disease that the user develops with high possibility and determines, for the user, as an individual examination item, an examination item for detecting presence or absence of the estimated disease, a common examination item determination unit that determines an examination item for detecting a disease having a high incidence rate in a first organization to which the user belongs or a disease having a high incidence rate in common in the first organization and a second organization having an attribute similar to an attribute of the first organization as a common examination item of people belonging to the first or second organization, and an examination item determination unit that determines an examination item of the user based on the individual examination item and the common examination item.

**Fig. 1**

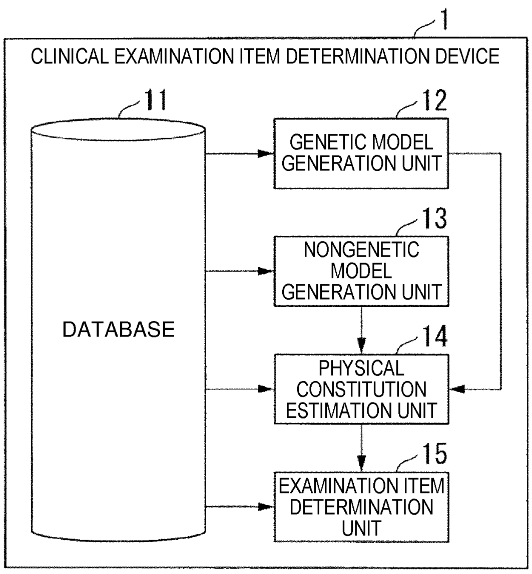


EP 4 109 457 A1

## Description

### Technical Field

The present invention relates to a technique for supporting human health management.

### Background Art

Conventionally, a periodical health examination such as a so-called medical checkup or thorough medical checkup has been performed for company employees under related laws and regulations. Examination items applied to an employee taking the periodical health examination (hereinafter referred to as "medical examinee") are basically often common examination items determined by a company that hires the medical examinee, although addition of examination items may be admitted as desired by the medical examinee. Therefore, in a conventional periodical health examination, because of a labor environment of a company, appropriate examination items are not always set about diseases that employees of the company are susceptible in common and diseases with individual differences in susceptibility to the diseases (see, for example, Non-Patent Literature 1). A service for proposing behaviors recommended to improve health (hereinafter referred to as "recommended behaviors") to a user and giving a fixed incentive to realization of the proposed behaviors by the user to promote health of the user (hereinafter referred to as "health service") has been in place (see, for example, Non-Patent Literature 2) .

### Citation List

### Non-Patent Literature

Non-Patent Literature 1: https://www.neckenpo.or.jp/member/hoken/fushime_ningen_do k.php, "Through Medical Checkup (turning point health examination)", NEC Kenpo NEC Corp. Health Insurance Society
Non-Patent Literature 2: https://health.dmkt-sp.jp/, d Health Care, NTT DOCOMO

### Summary of the Invention

### Technical Problem

However, in the conventional periodical health examination or health service, the examination items and the recommended behaviors are sometimes not set according to characteristics of the medical examinee or the user. Therefore, it is likely that the conventional periodical health examination or health service cannot sufficiently contribute to health improvement of the medical examinee or the user.
In view of the above circumstances, an object of the present invention is to provide a technique that can realize health management adapted to characteristics of an individual more.

### Means for Solving the Problem

An aspect of the present invention is a clinical examination item determination device that determines a clinical examination item for each user, the clinical examination item determination device including: an individual examination item determination unit that estimates, based on genetic factors of the user and nongenetic factors based on behaviors or habits of the user, a disease that the user develops with high possibility and determines, for the user, as an individual examination item, an examination item for detecting presence or absence of the estimated disease; a common examination item determination unit that determines an examination item for detecting a disease having a high incidence rate in a first organization to which the user belongs or a disease having a high incidence rate in common in the first organization and a second organization having an attribute similar to an attribute of the first organization as a common examination item of people belonging to the first or second organization; and an examination item determination unit that determines an examination item of the user based on the individual examination item and the common examination item.
An aspect of the present invention is a health behavior support device including: a recommended behavior setting unit that sets, based on genetic information of a user, for the user, a health behavior that is a behavior recommended to improve health of the user; and an application selection unit that selects, according to the health behavior set for the user, an application program executable in electronic equipment used by the user, the application program being an application program for supporting implementation of the health behavior by the user.

An aspect of the present invention is a clinical examination item determination method for determining a clinical examination item for each user, the clinical examination item determination method including: an individual examination item determination step for estimating, based on genetic factors of the user and nongenetic factors based on behaviors or habits of the user, a disease that the user develops with high possibility and determining, for the user, as an individual examination item, an examination item for detecting presence or absence of the estimated disease; an organization examination item determination step for determining an examination item for detecting a disease having a high incidence rate in a first organization to which the user belongs or a disease having a high incidence rate in common in the first organization and a second organization having an attribute similar to an attribute of the first organization as common examination items of people belonging to the first or second organization; and an examination item determination step for determining an examination item of the user based on the individual examination items and the common examination items.

An aspect of the present invention is a health behavior support method including: a recommended behavior setting step for setting, based on genetic information of a user, for the user, a health behavior that is a behavior recommended to improve health of the user; and an application determination step for determining, according to the health behavior set for the user, an application program executable in electronic equipment used by the user, a mission management application being an application program for supporting implementation of the health behavior by the user.

An aspect of the present invention is a computer program for causing a computer to function as the above clinical examination item determination device.

An aspect of the present invention is a computer program for causing a computer to function as the above health behavior support device.

Effect of the Invention

According to the present invention, it is possible to provide a technique that can realize health management adapted to characteristics of an individual more.

Brief Description of Drawings

[Fig. 1] Fig. 1 is a diagram showing a specific example of a functional configuration of a clinical examination item determination device in a first embodiment.
[Fig. 2] Fig. 2 is a diagram showing a specific example of a generation method for a genetic model in the first embodiment.
[Fig. 3] Fig. 3 is a diagram showing a relation among SNP1 of an alcohol dehydrogenase (ADH), SNP2 of an acetaldehyde dehydrogenase ALDH2, and a drinking or smoking lifestyle habit concerning development of esophageal cancer.
[Fig. 4] Fig. 4 is a diagram showing a specific example of a generation method for a nongenetic model in the first embodiment.
[Fig. 5] Fig. 5 is a diagram showing a specific example of an estimation method for a physical constitution in the first embodiment.
[Fig. 6] Fig. 6 is a first diagram for explaining a determination method for examination items in the first embodiment.
[Fig. 7] Fig. 7 is a second diagram for explaining the determination method for examination items in the first embodiment.
[Fig. 8] Fig. 8 is a third diagram for explaining the determination method for examination items in the first embodiment.
[Fig. 9] Fig. 9 is a block diagram showing a specific example of a functional configuration of a health behavior support device in a second embodiment.
[Fig. 10] Fig. 10 is a diagram showing a specific example of a setting method for a mission in a second embodiment.
[Fig. 11] Fig. 11 is a diagram showing a specific example of a selection method for a health application in the second embodiment.
[Fig. 12] Fig. 12 is a flowchart showing a specific example of a determination method for an application utilization degree in the second embodiment.
[Fig. 13] Fig. 13 is a flowchart showing a specific example of a determination method for behavior content in the second embodiment.

Description of Embodiments

Embodiments of the present invention are explained in detail with reference to the drawings.

<First Embodiment>

Fig. 1 is a diagram showing a specific example of a functional configuration of a clinical examination item determination device in a first embodiment. A clinical examination item determination device 1 (laboratory test item determination device) is a device that supports, based on a physical constitution or a belonging organization of a user, determination of items of a clinical examination (hereinafter referred to as "examination items") implemented on the user. The clinical examination item determination device 1 includes a CPU (Central Processing Unit), a memory, and an auxiliary storage device connected by a bus and executes a program. The clinical examination item determination device 1 functions as a device including a database 11, a genetic model generation unit 12, a nongenetic model generation unit 13, a physical constitution estimation unit 14, and an examination item determination unit 15 according to the execution of the program. Note that all or a part of the functions of the clinical examination item determination device 1 may be realized using hardware such as an ASIC (Application Specific Integrated Circuit), a PLD (Programmable Logic Device), or an FPGA (Field Programmable Gate Array). The program may be recorded in a computer-readable recording medium. The computer-readable recording medium is a portable medium such as a flexible disk, a magneto-optical disk, a ROM, or a CD-ROM or a storage device such as a hard disk incorporated in a computer system. The program may be transmitted via an electric communication line.

The database 11 is a database that stores various kinds of information necessary for determining examination items of users. The database 11 performs, according to requests of other functional units, registration of various data, deletion of registered data, and provision of requested data.

The genetic model generation unit 12 has a function of generating genetic models of the users. The genetic model is a model for estimating, based on factors due to genetic characteristics (hereinafter referred to as "genetic factors") of a user, possibility of the user developing a disease (intending a lifetime cumulative affection risk but, for convenience, hereinafter referred to as "incidence rate"). Specifically, the genetic model generation unit 12 generates, as a genetic model, an estimation model for outputting estimation values of incidence rates of diseases using genetic information of the user as an input. Note that it is assumed that the incidence rate estimated by the genetic model generation unit 12 is basically estimated based on the genetic factors of the user and the influence due to nongenetic factors of the user is not considered.

For the estimation of the incidence rate, information indicating a result of a clinical examination that the user took in the past in a periodical health examination or the like and information about lifestyle habits (hereinafter referred to as "information such as health examination history" may be further used. In this case, the genetic model generation unit 12 may generate a genetic model using the information such as health examination history as an input in addition to the genetic information. When a user having a predetermined gene has a predetermined lifestyle habit (smoking or the like), an incidence rate is sometimes extremely high. It is possible to improve accuracy of the incidence rate by considering such a combination of the genetic information and the information such as health examination history.

The genetic model generation unit 12 may generate, separately from the genetic model, an estimation model for outputting an incidence rate (hereinafter referred to as "health examination history model) using information concerning a health examination history as an input. Note that, when the health examination history model is generated separately from the genetic model, an incidence rate of diseases may be determined based on incidence rates estimated by both the models. For example, the incidence rate of the diseases may be determined as a weighted sum of an incidence rate estimated based on the genetic model and an incidence rate estimated based on the health examination history model. It is possible to improve estimation accuracy of an incidence rate even when a degree of a correlation between an incidence rate by a gene and an incidence rate by the information such as health examination history is unclear.

The nongenetic model generation unit 13 has a function of generating a nongenetic model of the user. The nongenetic model is a model for estimating an incidence rate of a disease due to factors other than genetic factors (hereinafter referred to as "nongenetic factors") of the user. For example, the nongenetic factors include physical characteristics, lifestyle habits, age, and a health examination result of the user. Note that the lifestyle habits include lifestyle habits of an individual such as drinking and smoking.

The incidence rate of the disease sometimes changes according to, in addition to the physical characteristics and the lifestyle habits of the individual user explained above, a physical burden, restriction of a behavior, or the like that the user receives in activities performed in a belonging organization such as a company. For example, in an organization where deskwork is mainly performed, it is assumed that an exercise amount of the user is highly likely to be lower than a general average and the user easily suffers from a lifestyle disease due to shortage of exercise. Therefore, factors based on activities of the user in the belonging organization may be included in the lifestyle habits serving as the nongenetic factors.

For example, concerning the lifestyle diseases due to the shortage of exercise explained above, an indicator value of an exercise amount measured for each user may be included in the nongenetic factors. When it is difficult to measure the indicator value of the exercise amount for each user, information correlating with the exercise amount may be included in the nongenetic factors. For example, when the exercise amount of the user is considered to be different depending on a job type, job types of users may be included in the nongenetic factors. For example, when the exercise amount of the user is considered to be different depending on a belonging organization, belonging organizations of the users may be included in the nongenetic factors. In this way, any direct factors or indirect factors considered to correlate with the incidence rate of the disease can be included in the nongenetic factors.

Note that the nongenetic model does not always need to be configured as one estimation model and may be configured to estimate a final incidence rate based on estimation results of a plurality of estimation models based on different nongenetic factors or a combination of the different nongenetic factors. The nongenetic factors may be set based on data of a single year or may be set based on data of a plurality of years. By inputting the nongenetic factors based on the data of the plurality of years, it is possible to generate a nongenetic model considering a changeover years of the nongenetic factors.

The physical constitution estimation unit 14 has a function of estimating a physical constitution of the user based on a genetic model and/or a nongenetic model of the user. Note that the physical constitution of the user means an incidence rate of a disease estimated from genetic factors and/or nongenetic factors of the user. Note that genetic characteristics of the user and nongenetic factors of the user sometimes mutually affect a disease risk. For example, a user having a predetermined gene polymorphism easily develops lung cancer when the user smokes. In such a case, a weighted sum of disease risks estimated by two models as explained below may be calculated or one model may be configured based on both of genetic characteristics of the user and nongenetic characteristic of the user.

The examination item determination unit 15 has a function of determining examination items implemented on the user. Specifically, the examination item determination unit 15 determines examination items of a disease having a high incidence rate in an organization such as a company to which the user belongs (a first organization) or examination items of a disease having a high incidence rate in common in the organization and an organization having an attribute similar to an attribute of the organization (a second organization) (hereinafter referred to as "common examination items") and individual examination items for each user (hereinafter referred to as "individual examination items"). The examination item determination unit 15 determines the common examination items based on probabilities that, in the organization to which the user belongs, members of the organization develop various diseases and determines the individual examination items based on the physical constitution of the user estimated by the physical constitution estimation unit 14.

By including such a configuration, the clinical examination item determination device 1 in the first embodiment can include, according to the organization to which the user belongs and the physical constitution of the user, in examination items of the user, an examination item contributing to detection of a disease that the user develops with high possibility. In the following explanation, a generation method for a genetic model and a nongenetic model, a method of estimating a physical constitution of the user, and a method of determining examination items of the user are explained in detail.

Fig. 2 is a diagram showing a specific example of a generation method for a genetic model in the first embodiment. A case control related analysis or the like can be used for generation of a genetic model. In the case control related analysis, about a relation between a disease and SNP (single-nucleotide polymorphisms), a group having diseases and a group not having diseases are statistically compared to determine whether there is a difference in frequencies of a gene polymorphism. The relation is considered to be stronger as an obtained P value (a probability of accidental occurrence) is lower (see, for example, reference document 1).

It is known that a relation of the SNP to a certain decease is found by a study using a GWAS (Genome-Wide Association Study). Specifically, as information concerning a risk allele (R) and a non-risk allele (N) of the SNP, an odds ratio r1 of a risk hetero (RN) to a non-risk homo (NN) and an odds ratio r2 of a risk homo (RR) to a non-risk homo (NN) are obtained (see, for example, reference document 3).

Fig. 2 is a table summarizing the numbers of samples of gene types in a case and a control in the GWAS. The "control" indicates the number of samples in a group not developing a disease and the "case" indicates the number of samples of a group developing a disease. In this case, the odds ratios r1 and r2 are obtained from the following Expression (1).

[Math. 1]

$$r_1 = \frac{A \cdot E}{B \cdot D}, \quad r_2 = \frac{A \cdot F}{C \cdot D} \quad \cdots (1)$$

About the gene types (NN, RN, and RR) of the SNP, a frequency of appearance of the risk allele R is represented as p (that is, a frequency of appearance of the non-risk allele N is 1-p), an overall incidence rate is represented as q, and incidence rates in the respective gene types are represented as d1, d2, and d3. The incidence rates d1 to d3 of the gene types are obtained by solving a relational expression of these. In this case, the overall incidence rate q about the single SNP is represented by the following Expression (2). It is possible to specify an incidence rate according to which gene type the single SNP has.

[Math. 2]

$$q = d_1 (1-p)^2 + 2d_2 p(1-p) + d_3 p^2 \quad \cdots (2)$$

When a plurality of SNPs are related, it is possible to represent a comprehensive incidence rate P with, for example, Expression (3) by using a multiplication model of incidence rates of gene types of the respective SNPs.

[Math. 3]

$$P = q \prod_{i=1}^{n} \left( \frac{d_i}{q} \right) \quad \cdots (3)$$

Note that it is assumed that genetic information of users used for the generation method is registered in the database 11 in advance as one kind of user information. Creation of the genetic model is not limited to the method explained above. For example, the genetic model may be created using a polygenic risk score (see, for example, reference document 2) or the like.

As explained above, the influence due to the nongenetic factors of the user is not basically considered for the disease risk estimated by the genetic model generation unit 12. This is because the influence on an incidence rate of a disease by the nongenetic factors of the user is taken into account by a nongenetic model explained below. However, it is known that an estimation result of a disease risk based on the SNP greatly fluctuates according to a combination with the nongenetic factors of the user (see, for example, reference document 4). For example, Fig. 3 is a diagram showing a relation among SNP1 of an alcohol dehydrogenase (ADH), SNP2 of an acetaldehyde dehydrogenase ALDH2, and a drinking or smoking lifestyle habit concerning development of esophageal cancer. As it is seen from Fig. 3, a disease risk of a user having the drinking or smoking lifestyle habit is extremely larger than the disease risk estimated based on the SNP. Therefore, the genetic model generation unit 12 may be configured to estimate a disease risk according to a combination of the SNP and the nongenetic factors of the user in order to estimate a more accurate disease risk.

The genetic model generation unit 12 registers information indicating a correlation between the disease and the genetic information created based on such a method in a database as a genetic model. Up to this point, a relation between the genetic information and the disease rink is considered to be successfully modeled. By inputting genetic information of a target user, whose disease risk is estimated, to the genetic model obtained in this way, it is possible to estimate a disease risk of the user. Construction of a genetic model and estimation using the genetic model may be separately performed.

Fig. 4 is a diagram showing a specific example of a nongenetic model in the first embodiment. For example, the nongenetic model generation unit 13 in the first embodiment generates a nongenetic model for estimating a physical constitution of a user using a method of machine learning. For example, Fig. 4(A) shows, as an example of a learning model by the machine learning, a learning method in the case in which a neural network is used. Presence or absence and a sign of a disease often appear in a result of a health examination. A tendency of development of a disease greatly depends on nongenetic factors of a person having the disease. Therefore, the nongenetic model generation unit 13 learns a relationship between human nongenetic factors (parameters 1 to N) and incidence rates (Y1 to YM) of diseases.

Specifically, associated information of information indicating nongenetic factors (explanatory variables) and information indicating incidence rates (objective variables) of diseases in a group having the nongenetic factors is prepared as one set of sample data. A set of the sample data acquired for each of classifications of the nongenetic factors is prepared as learning data. The nongenetic model generation unit 13 applies the learning data prepared in this way to the learning model of the neural network to generate, as a learned model, a neural network describing a relationship between the explanatory variables and the objective variables. The nongenetic model generation unit 13 outputs the learned model generated in this way to the physical constitution estimation unit 14. By inputting nongenetic information of the user to the nongenetic model (the learned model) generated in this way, it is possible to estimate incidence rates of diseases about the user. Note that the method of the machine learning is not limited to the above neural network. For example, for the method of the machine learning, another learning model such as a GP (Genetic Programming) may be used or a statistical analysis method such as a multivariate analysis may be used.

Any examination results that can be measured by a physical examination such as a result of a blood test, a result of a urine test, a result of an X-ray test, and a measurement result of an electrocardiogram can be included in a health examination result that could be included in the nongenetic information. Height and weight, a value of BMI (Body Mass Index), an abdominal circumference, blood pressure, sex, and the like can be included in physical characteristics that could be included in the nongenetic information. Various kinds of information such as a smoking history and a smoking amount, a drinking history and a drinking amount, an average wakeup time, an average bedtime, meal time periods, an exercise time in one day, and a work situation can be included in lifestyle habits that could be included in the nongenetic information. Any information indicating characteristics and amounts of work such as average work start time and average work end time, an average overtime, the number of days of holiday shift, a rate of deskwork in the work, a type of a job, and a belonging organization and characteristics of the organization can be included in the information indicating the work situation.

Fig. 5 is a diagram showing a specific example of an estimation method for a physical constitution in the first embodiment. Specifically, the physical constitution estimation unit 14 calculates a weighted sum of incidence rates of diseases respectively obtained by the genetic model generated by the genetic model generation unit 12 and the non-genetic model generated by the nongenetic model generation unit 13 to thereby estimate incidence rates about diseases of the user. The incidence rates of the diseases based on the genetic model are obtained by comparing the genetic information and the genetic model (indicating a correlation between the genetic information and the incidence rates) of the user. When a gene polymorphism having a high correlation with a disease is included in the genetic information of the user, a high incidence rate is estimated about the disease. On the other hand, the incidence rates of the diseases based on the nongenetic model are obtained by inputting parameters indicating the nongenetic factors of the user to the learned model. For example, an incidence rate of a disease is calculated by the following Expression (4).

[Math. 4]

$$\text{Incidence rate} = \alpha \times \text{the incidence rate estimated by the genetic model} + (1-\alpha) \times \text{the incidence rate estimated by the nongenetic model} \quad \cdots (4)$$

In the expression, $\alpha$ is a coefficient for weighting the influences of the genetic model and the nongenetic model ($0 \leq \alpha \leq 1$). A value of $\alpha$ may be determined based on any determination standard. For example, the value of $\alpha$ may be determined as a fixed value or may be determined as a variable value based on the following idea. For example, since the genetic factors are considered to less easily change, the genetic factors are considered to be a base of physical constitution estimation irrespective of the age of the user. On the other hand, since the nongenetic factors are considered to easily change according to timing and elapse of time, the nongenetic factors are considered to take into account factors corresponding to timing with respect to the base of the physical constitution estimation based on the genetic factors. For example, habitual drinking and smoking are examples of the nongenetic factors. These lifestyle habits tend to increase an incidence rate of a disease as age is higher. In this case, it is conceivable to reduce a value of $\alpha$ as the age is higher such that the influence on an incidence rate by the nongenetic factors increases as the age is higher.

On the other hand, it is also possible to assume that a child is easily affected by lifestyle habits of parents but, when growing up, selects an environment matching characteristics of the individual, whereby the influence on an incidence rate by genetic characteristics tends to be amplified. In this case, it is conceivable to increase the value of $\alpha$ as the age is higher. Depending on a disease, a degree of the influence of the nongenetic factors on an incidence rate of the disease is different. In this case, the value of $\alpha$ may be determined for each disease. In this way, the value of $\alpha$ may be represented by a function that changes according to age and a type of a disease (see, for example, reference document 5). The physical constitution estimation unit 14 can determine, as an individual examination item, an examination item of a disease having an incidence rate exceeding a threshold, among diseases, incidence rates of which are calculated in this way.

Note that the incidence rate may be represented by a relational expression other than Expression (4). For example, the incidence rate may be represented not by a linear form like Expression (4) but by a nonlinear form. For example, the incidence rate may be calculated by a relational expression for not determining a final incidence rate based on the incidence rate estimated based on the genetic factors and the incidence rate estimated based on the nongenetic factors like Expression (4) but for directly estimating a final incidence rate simultaneously considering the influences of the genetic factors and the nongenetic factors.

For example, the incidence rate may be calculated by correcting, based on a result of a case control study concerning the nongenetic factors, the incidence rate estimated based on the genetic factors. For example, it is assumed that, about a disease for which an incidence rate of approximately six times as high as a normal incidence rate is assumed by a specific genetic factor, experiment data indicating that the incidence rate increases to 190 times because specific lifestyle habits of drinking and smoking equal to or more than predetermined amounts are added to the specific genetic factor as nongenetic factors is obtained. In such a case, the physical constitution estimation unit 14 may be configured to correct, according to whether the nongenetic factors of the user correspond to the experiment data, the incidence rate of the user having the above specific genetic factor.

Fig. 6 to Fig. 8 are diagrams for explaining a determination method for examination items in the first embodiment. Fig. 6 and Fig. 7 among Fig. 6 to Fig. 8 are diagrams showing a specific example of a determination method for common examination items. First, the examination item determination unit 15 acquires organization information and population information from the database 11. As explained above, the organization information is information indicating incidence rates of diseases in the organization to which the user belongs. The population information is information indicating incidence rates of diseases in a population to which the user belongs. It is assumed that the organization information and the population information are registered in the database 11 in advance as information indicating a result of an epidemiological survey for the organization and the population.

The population is a group larger in size than the organization to which the user belongs. For example, the population information is information indicating incidence rates of diseases in a region (a country, a prefecture, a municipality, or the like) where the user lives. The examination item determination unit 15 extracts, based on the organization information and the population information, a disease having an incidence rate in the organization higher than a general incidence rate in the population and determines, as common examination items of the user, examination items effective for detection of the extracted disease.

For example, according to the following Expression (5), the examination item determination unit 15 calculates indicator values of susceptibility to diseases in the organization and extracts a disease having the indicator value exceeding a predetermined threshold. Alternatively, the examination item determination unit 15 may select a predetermined number of diseases in order from a disease having the highest indicator value.

[Math. 5]

$$\text{Indicator value} = \text{the incidence rate in the belonging organization/the incidence rate in the population} \quad \cdots (5)$$

Note that a standard deviation or the like of the incidence rate in the belonging organization with respect to the incidence rate in the population may be used as the indicator value. It is assumed that information indicating effective examination items effective for the diseases is registered in the database 11 in advance.

Note that the examination item determination unit 15 may be configured to consider medical expenses in the determination of the common examination items. For example, it is assumed that the incidence rates of the diseases are set as shown in Fig. 7(A) and expenses (medical expenses) required for treatment of the diseases are set as shown in Fig. 7(B). In this case, the examination item determination unit 15 may calculate a weighted sum of the incidence rates and the medical expenses as an indicator value as indicated by the following Expression (6). Note that Expression (6) is an example of a calculation formula for an indicator value in the case in which a target disease is "gingivitis/periodontal disease".

[Math. 6]

$$\text{Indicator value} = \beta \times R1 + \gamma \times C1 \quad \cdots (6)$$

It is assumed that the "medical expenses" shown in Fig. 7(B) are values normalized to calculate the weighted sum of the medical expenses and the incidence rates. For example, the values of the "medical expenses" are values obtained by dividing the medical expenses of the diseases by the largest medical expense among the medical expenses of all the diseases. $\beta$ and $\gamma$ in Expression (6) are coefficients for adjusting weights of the incidence rates and the medical expenses. $\beta$ and $\gamma$ may be optionally determined according to which degree of weight is put on which of the incidence rates and the medical expenses.

About diseases described below, the examination item determination unit 15 may include examination items of the diseases in the common examination items without performing comparison with the population.

Diseases included in receipts issued in the past to the belonging organization of the user or diseases, the number of suffers of which is large, among those diseases. By including such diseases in the common examination items, it is possible to preferentially set examination items of a disease easily developed in the belonging organization.

Diseases, incidence rates of which are estimated as high based on an epidemiological survey result of the Ministry of Health, Labor and Welfare and average age of the organization. This is because the incidence rates of the diseases in the entire organization tend to increase according to aging in the organization. For example, when the average age of the organization is set as an indicator and the indicator exceeds a threshold based on a result of an epidemiological survey, the examination item determination unit 15 may include examination items of a target disease of the epidemiological survey in the common examination items.

Diseases determined as having high possibility of development based on statistical values of incidence rates estimated from genetic models or nongenetic models of members of the organization. In this way, data mainly used for determining the individual examination items may be used for the determination of the common examination items. In particular, nongenetic elements are considered to be highly likely to be similar among the members of the belonging organization.

Fig. 8 is a diagram showing a specific example of a determination method for individual examination items in the first embodiment. In general, possibility of development of some disease is different depending on age (see, for example, reference document 6). Therefore, according to the age of the user, it is desirable to perform an examination about a disease having high possibility of development in the age and does not perform an examination about a disease having low possibility of development to suppress an increase of examination expenses. Therefore, first, about diseases estimated having relatively high incidence rates by the physical constitution estimation unit 14, the examination item determination unit 15 acquires, based on a result of an epidemiological survey, age in which the diseases are easily developed (hereinafter referred to as "onset age").

Fig. 8 is a diagram showing a result of an epidemiological survey used in specifying the age (see, for example, reference document 7). Specifically, Fig. 8 is a diagram showing a survey result of transitions of incidence rates due to aging of laryngeal cancer and pharyngeal cancer. As it is seen from Fig. 8, it is seen that, about the laryngeal cancer and the pharyngeal cancer, the incidence rate suddenly increases from near 45 years old in male and the incidence rate gradually increases from near 30 years old in female. It is assumed that a result of an epidemiological survey about such diseases is registered in advance in the database 11. In this case, the examination item determination unit 15 can designate a target disease and acquire a survey result of the disease from the database 11 and specify, as onset age, age in which a gradient of an incidence rate curve indicated by the survey result exceeds a predetermined threshold.

Note that, information indicating onset ages of diseases specified based on the result of the epidemiological survey may be registered in advance in the database 11. In this case, by designating a target disease, the examination item determination unit 15 can acquire onset age of the disease from the database 11.

The examination item determination unit 15 extracts, based on the onset ages of the diseases acquired in this way, about a disease, onset age of which exceeds the age of the user, examination items effective for detection of the disease as individual examination items. For example, in the example shown in Fig. 8, the examination item determination unit 15 includes examination items of laryngeal cancer and pharyngeal cancer in the individual examination items when the age of a male user is fifty years old and excludes the examination items from the individual examination items when the age is forty years old.

Note that, about a disease for which a relatively high incidence rate is estimated among the diseases, the incidence rates of which are estimated by the physical constitution estimation unit 14, the examination item determination unit 15 may include examination items of the disease in the individual examination items irrespective of the age of the user. In this case, a threshold of an incidence rate only has to be determined in advance as a reference in not performing selection of a disease by age. The examination item determination unit 15 outputs, as examination items of the user, the common examination items and the individual examination items determined in this way.

The clinical examination item determination device 1 in the first embodiment configured in this way can determine items of a clinical examination performed on the user from both aspects of the possibility of development of the disease based on the organization to which the user belongs and the possibility of development of the disease based on the genetic factors or/and the nongenetic factors of the user. The examination items determined in this way are proposed to the user or the belonging organization of the user, whereby it is possible to promote health management adapted to characteristics of an individual more. Note that the items of the clinical examination may be determined independently using an incidence rate of the disease based on each of the organization to which the user belongs, the genetic factors of the user, and the nongenetic factors of the user or the items of the clinical examination may be determined by combining two or all of incidence rates of the disease based on each of the organization to which the user belongs, the genetic factors of the user, and the nongenetic factors of the user.

Sources of the reference documents introduced in the first embodiment are described below.

Reference document 1: http://www.riken.jp/pr/press/2010/20100212/, "A gene 'ACACB' relating to diabetic nephropathy is found".

Reference document 2: https://www.rgare.com/docs/default-source/subsite-materials/japan-reflections/reflections-vol-45.pdf, "Polygene risk score PRS: Integrate thousands of gene mutations to predict a disease".

Reference document 3: https://mycode.jp/benefits/basis.html, "Quality of scientific grounds".

Reference document 4: https://biobankjp.org/cohort_1st/public/tsushin07/biobank _tsushin07_02.html, "Approach to a relation between liquor and cigarette and esophageal cancer".

Reference document 5: https://dot.asahi.com/aera/2019072400072.html?page=1, "Influence of inheritance is 87% for mathematics, 66% for IQ, and 59% for income! What are astonishing latest research results?".

Reference document 6: https://ganjoho.jp/reg_stat/statistics/stat/summary.html, "Latest cancer statistics".

Reference document 7: Cancer Information Services, National Cancer Center, Japan "Cancer registration/statistics".

Note that, in this embodiment, the configuration for determining examination items of a user based on both of a physical constitution and a belonging organization of the user is explained. However, the clinical examination item determination device 1 in the embodiment may be configured to determine examination items based on one of the physical constitution and the belonging organization.

The clinical examination item determination device 1 may be configured to include an individual examination item determination unit that determines individual examination items out of predetermined examination items and a common examination item determination unit that determines common examination items out of the predetermined examination items. In this case, the examination item determination unit 15 may be configured to determine examination items of users based on the individual examination items determined by the individual examination item determination unit and the common examination items determined by the common examination item determination unit.

About the disease for which high possibility of development is estimated according to both of the incidence rate and the result of the epidemiological survey, the clinical examination item determination device 1 in the first embodiment selects the examination items of the disease as the individual examination items. This is considered to be a method of estimating high possibility of development under an AND condition of a viewpoint of the incidence rate and a viewpoint of the result of the epidemiological survey. In contrast, about a disease for which high possibility of development is estimated according to each of the incidence rate and the result of the epidemiological survey, the clinical examination item determination device 1 may be configured to select examination items of the disease as the individual examination items. Specifically, first, the clinical examination item determination device 1 selects, based on the result of the epidemiological survey, diseases, onset ages of which are exceeded by the age of the user, and selects examination items of the diseases as the individual examination items. In addition, the clinical examination item determination device 1 selects diseases having high incidence rates based on genetic information of the user and selects examination items of the diseases as the individual examination items. In this way, the clinical examination item determination device 1 is capable of selecting more examination items under an OR condition of the viewpoint of the incidence rate and the viewpoint of the result of the epidemiological survey. It is possible to perform an examination without omission covering examination items corresponding to age and examination items having individual differences.

The clinical examination item determination device 1 may have a liquid biopsy as one of choices of examination items. The liquid biopsy is an examination method attracting attention in recent years as being capable of diagnosing a plurality of types of cancer at a time. However, in the liquid biopsy, a type of cancer that can be detected is different depending on a method of the liquid biopsy. Therefore, the clinical examination item determination device 1 may be configured to select, as an examination item, the liquid biopsy corresponding to cancer estimated as having a high incidence rate. Consequently, it is possible to determine a method that can efficiently and effectively examine cancer.

<Second Embodiment>

Fig. 9 is a block diagram showing a specific example of a functional configuration of a health behavior support device 2 (health behavior support device) in a second embodiment. Whereas the clinical examination item determination device 1 in the first embodiment has the function of determining items of a clinical examination implemented for a user, the health behavior support device 2 in the second embodiment is different from the clinical examination item determination device 1 in the first embodiment in that the health behavior support device 2 has a function of supporting a behavior performed by a user to promote health (hereinafter referred to as "health behavior"). The health behavior support device 2 includes a CPU, a memory, an auxiliary storage device, and the like connected by a bus and executes a program. The health behavior support device 2 functions as a device including a database 21, a mission setting unit 22, a mission management application selection unit 23, an application utilization degree determination unit 24, and a behavior content determination unit 25. The health behavior support device 2 is communicably connected to user terminals 3 used by users. The user terminal 3 is electronic equipment capable of executing a health application explained below. For example, the user terminal 3 is an information processing device such as a smartphone, a cellular phone, a tablet terminal, or a personal computer.

All or a part of the functions of the health behavior support device 2 may be realized using hardware such as an ASIC (Application Specific Integrated Circuit), a PLD (Programmable Logic Device), or an FPGA (Field Programmable Gate Array). The program may be recorded in a computer-readable recording medium. The computer-readable recording medium is a portable medium such as a flexible disk, a magneto-optical disk, a ROM, or a CD-ROM or a storage device such as a hard disk incorporated in a computer system. The program may be transmitted via an electric communication line.

The database 21 is a database storing various kinds of information necessary for supporting health behaviors of users. The database 21 performs, according to requests from the other functional units, registration of various data, deletion of registered data, and provision of requested data.

The mission setting unit 22 (a recommended behavior setting unit) has a function of setting, for the users, health behaviors recommended according to genetic constitutions of the respective users. In the following explanation, the health behaviors set for the users are referred to as "missions". The users install, in the user terminals 3, application programs corresponding to the missions set for the users (hereinafter referred to as "health applications") and manage the missions with the health applications. The health applications may give, to the users who have achieved the missions, fixed incentives corresponding to the missions.

The mission management application selection unit 23 selects, for the users for whom the missions are set by the mission setting unit 22, health applications for supporting management of the set missions (hereinafter referred to as "mission management applications"). The mission management application selection unit 23 encourages the users to install the health applications selected as the mission management applications in the user terminals 3. Note that the mission management application selection unit 23 may only notify the health applications selected as the mission management applications to the user terminals 3 and leave it to determination by the users whether to install the health applications.

The application utilization degree determination unit 24 has a function of determining utilization degrees of the health applications installed in the user terminals 3 as the mission management applications. The utilization degrees of the mission management applications are indicators for measuring utilization degrees of the users for the set missions. The application utilization degree determination unit 24 plays a role of performing notification corresponding to the determined application utilization degrees to the user terminals 3 to thereby improve motivations of the users to execute health behaviors for the set missions.

The behavior content determination unit 25 has a function of determining content of health behaviors actually performed by the users. Specifically, the behavior content determination unit 25 acquires, from the health applications, information concerning the health behaviors performed by the users from the user terminals 3 (hereinafter referred to as "behavior information") and determines appropriateness of the performed health behaviors based on the acquired behavior information. The behavior content determination unit 25 performs, to the user terminal 3, notification corresponding to the appropriateness of the health behavior performed by the user of the user terminal 3. Consequently, the behavior content determination unit 25 plays a role of improving the quality of the health behavior performed by the user and improving a motivation of the user to execute a higher quality health behavior.

Fig. 10 is a diagram showing a specific example of a setting method for a mission in the second embodiment. First, the mission setting unit 22 acquires genetic information of the user from the database 21 and specifies a gene of the user based on the acquired genetic information. The mission setting unit 22 selects a mission for promoting health of the user having a physical constitution indicated by the specified gene and notifies the selected mission to the mission management application selection unit 23. Note that it is assumed that genetic information of the users is registered in the database 21 in advance.

Note that, when information such as a medical questionnaire is acquired beforehand, the mission setting unit 22 may be configured to filter selected missions based on information concerning the missions. For example, it is likely that a health behavior about to be set as a mission has already been implemented by the user. In such a case, if the health behavior already implemented by the user can be grasped according to the information such as the medical questionnaire, it is possible to avoid such a health behavior unnecessary for the user being set as a mission. For example, it is possible to prevent a health behavior of "refraining from smoking" from being set for a user having a risk of lung cancer but not having a smoking history. Such filtering achieves an effect of enabling a user, to whom many health behaviors are recommended as missions, to more easily grasp the recommended health behaviors.

Specifically, genetic constitution information shown in Fig. 10 is registered in the database 21 in advance. The genetic constitution information is information indicating a correspondence relation between a physical constitution indicated by a specific gene polymorphism and a health behavior desired to be executed for the physical constitution. For example, the genetic constitution information of the example shown in Fig. 10 indicates that a person having a gene polymorphism of $\beta$3AR tends to be weak in a function of carbohydrate metabolism and, therefore, the person desirably actively takes vitamin B1 for promoting the carbohydrate metabolism. When, according to the acquired genetic information, the user has any gene polymorphism included in the genetic constitution information, the mission setting unit 22 sets a health behavior associated with the gene polymorphism as a mission of the user. For setting of a health application in a later stage, the mission setting unit 22 notifies a behavior classification of the health behavior to the mission management application selection unit 23 together with the set mission.

Note that the physical constitution indicated by the gene polymorphism is not limited to the physical constitution illustrated in Fig. 10. The genetic constitution information is desirably updated at any time with reference to a study result or the like concerning a relation between a gene polymorphism and a physical constitution. For example, when a relationship between physical constitutions concerning life such as resistance against a labor burden, the quality of sleep, and affinity with worn clothes and gene polymorphisms is clarified by a study in future, health behaviors for promoting health with respect to these physical constitutions are desirably added to the genetic constitution information.

Fig. 11 is a diagram showing a specific example of a selection method for a health application in the second embodiment. First, the mission management application selection unit 23 acquires correspondence information indicating a correspondence relation between behavior classifications and health applications from the database 21 and selects, based on the acquired correspondence information, as a mission management application, a health application corresponding to a behavior classification notified from the mission setting unit 22. The mission management application selection unit 23 encourages the user to install the selected mission management application in the user terminal 3. Note that it is assumed that the correspondence information is registered in the database 21 in advance. In the following explanation, as a specific example of the health application selected as the mission management application, a meal management application and an exercise support application illustrated in Fig. 11 are explained.

(1) Meal management application

As explained above, the person having the gene polymorphism "β3AR" tends to be weak in the function of carbohydrate metabolism. Such a physical constitution is a physical constitution having a risk of easily gaining visceral fat (see, for example, reference document 7). Therefore, for the person having such a physical constitution, a health behavior for taking more vitamin B1 for supporting the carbohydrate metabolism than a standard is set as a mission. Accordingly, as a mission management application corresponding to such a mission, a meal management application having a function that can evaluate an intake amount of vitamin B1 is selected. For example, the meal management application has a function of analyzing image data obtained by imaging food eaten by the user, estimating an amount of vitamin B1 included in the food, and transmitting an estimated value of the amount to the health behavior support device 2. Note that the meal management application may be configured to transmit the image data to a server having an analysis function for the image data and acquire an analysis result of the image data from the server.

(2) Exercise support application

A person having an S/S type of a gene polymorphism "PPARGC1A" tends to have a small increase amount of mitochondria by exercise and to be weak in a function of energy production by exercise. Such a physical constitution is a physical constitution not suitable for exercise with a large energy consumption amount requiring durability (see, for example, reference document 8). Therefore, for the person having such a physical constitution, a health behavior for performing light exercise such as walking for a long time is set as a mission. As a mission management application for such a mission, an exercise support application having a function that can evaluate a time of walking is selected. For example, the exercise support application has a function of acquiring measurement information of an activity amount from an activity amount meter worn by the user and transmitting the acquired measurement information to the health behavior support device 2.

Note that a health behavior that could be a mission is not limited to the health behavior illustrated in Fig. 10. In the following explanation, several health behaviors that could be missions other than the health behaviors illustrated in Fig. 10 are enumerated from the viewpoints of exercise, weight, drinking, smoking, sleep, lifestyle rhythm, meal, beverage other than alcohol (for example, coffee or tea).

[Exercise]

Walk one hour or more a day
Walk seven hours or more a week

[Weight]

Not excessively increase weight
Not excessively lose weight

[Drinking]

Refrain from drinking heavily
Refrain from drinking beer
Refrain from drinking wine

[Coffee]

Drink coffee every day
Not excessively drink coffee

[Tea]

Not excessively drink green tea
Drink green tea
Refrain from drinking hot tea

[Cigarette]

Not smoke cigarettes
Refrain from cigarettes immediately after wakeup

[Sleep]

Not excessively reduce a sleep time
Not excessively increase a sleep time

[Lifestyle rhythm]

Not work excessively long
Avoid night shift
Avoid shift work

[Meal]

Not excessively eat orange
Take calcium and vitamin D
Eat a lot of tomatoes
Eat nuts, fish, fruits and vegetables
Take a lot of vitamin C
Refrain from fat
Take magnesium in food
Refrain from soy beans and milk
Often eat soybeans
Refrain from carbohydrate
Often eat meat
Often take dairy products

Fig. 12 is a flowchart showing a specific example of a determination method for an application utilization degree in the second embodiment. First, the application utilization degree determination unit 24 acquires, from the mission management application installed in the user terminal 3, recommended frequency information of a mission management application selected for the user (step S101). The recommended frequency information is information indicating a frequency of use recommended about health applications (hereinafter referred to as "recommended use frequency"). The recommended use frequency is the number of times of use or a use time period of the mission management application in a predetermined unit period (for example, one day or one week). For example, the number of times of use can be the number of times the mission management application is started. The number of times of use may be the number of times a predetermined function is used in the mission management application. For example, the use time period can be a time in which the mission management application is started. The use time period may be a time in which the mission management application is operating in the foreground. Such a recommended use frequency is set in advance to a use frequency at which a health activity performed by the user to achieve a mission can be appropriately managed.

Note that it is assumed that the recommended frequency information is retained in health applications. However, the use frequency information may be registered in the database 21 in advance. The recommended use frequency may be set for each health application or may be set for each combination of a health application and a mission.

Subsequently, the application utilization degree determination unit 24 acquires, from the user terminal 3, use history information indicating a history of the user using the mission management application (step S102). The application utilization degree determination unit 24 acquires, based on the acquired use history information, a frequency of the user actually using the mission management application (hereinafter referred to as "actual use frequency") (step S103) and determines whether the actual use frequency is equal to or higher than the recommended use frequency (step S104). In order to compare the actual use frequency and the recommended use frequency, an actual number of times of use of the mission management application in a predetermined unit period or information with which the actual number of times of use can be grasped only has to be included in the use history information.

When the actual use frequency is lower than the recommended use frequency in step S104 (step S104 - NO), the application utilization degree determination unit 24 transmits, to the user terminal 3, a notification for urging further use of the mission management application (step S105). Note that, instead of transmitting the notification to the user terminal 3, the application utilization degree determination unit 24 may perform the notification with display on the health application or may perform the notification with transmission of an electronic mail. When a portal site for each user is provided as an example of a user interface provided by the health behavior support device 2 through a Web, the application utilization degree determination unit 24 may display the notification on screens generated by the users logging in to the portal site.

In this case, the application utilization degree determination unit 24 may notify information indicating how much the real use frequency is less than the recommended use frequency. On the other hand, when the actual use frequency is equal to or higher than the recommended use frequency in step S104 (step S104 - YES), the application utilization degree determination unit 24 gives a fixed point to the user as a reward for steadily executing the mission (step S106).

For example, when a mission concerning meals of the user is managed by the meal management application, it is conceivable to set "three times/one day" as the recommended use frequency to correspond to three meals in the morning, the noon, and the night. In this case, if the actual use frequency of the meal management application is three times or more/one day, the application utilization degree determination unit 24 gives a point to the user and transmits a message such as "Got a point!" to the user terminal 3. On the other hand, if the actual use frequency of the meal management application is zero times to twice/one day, the application utilization degree determination unit 24 transmits a message such as "Let's input data to the application!" to the user terminal 3 as a notification for urging further use of the meal management application.

Fig. 13 is a flowchart showing a specific example of a determination method for behavior content in the second embodiment. A method conceding the meal management application is explained as an example of the determination method for behavior content. In this example, it is assumed that the user registers image data obtained by imaging food eaten by the user (hereinafter referred to as "meal image data") in the meal management application in advance as behavior information. In this case, first, the behavior content determination unit 25 acquire the meal image data registered by the user from the user terminal 3 (step S201). The behavior content determination unit 25 analyzes the acquired meal image data to thereby estimate a calory amount and a nutrient amount taken by the user (step S202). For such an analysis of the meal image, for example, a technique described in reference document 9 can be used.

Subsequently, the behavior content determination unit 25 acquires information concerning the mission set for the user (hereinafter referred to as "mission information") (step S203). It is assumed that the mission information is registered in the meal management application or the database 21 of the user terminal 3 by the mission setting unit 22 when the mission is set for the user. Specifically, a health behavior set as the mission and a target value concerning the health behavior are included in the mission information.

For example, in the example shown in Fig. 10, when active intake of vitamin B1 is set as a mission for the user having the gene polymorphism "β3AR", the health behavior, a range of an intake amount of a nutrient (vitamin B1) and calory targeted in the health behavior (hereinafter referred to as "target range") are included in the mission information. Note that the target range may be defined in advance in correlation with the genetic constitution information illustrated in Fig. 10 or may be customized for each user in a combination with other physical constitutions of the user. Based on the acquired mission information, the behavior content determination unit 25 discriminates the mission set for the user (step S204) and specifies a target value of the intake amount of the calory and the nutrient set in the mission (step S205).

Subsequently, the behavior content determination unit 25 determines whether an intake amount of calory and the nutrient taken by the user (hereinafter simply referred to as "intake amount") is smaller than a lower limit of the target range (step S206). The intake amount is obtained by the analysis of the meal image data in step S202. When the intake amount is smaller than the lower limit value of the target range in step S206 (step S206- YES), the behavior content determination unit 25 notifies the user terminal 3 that the intake amount of the calory and the nutrient is insufficient (an example of an advice) (step S207).

On the other hand, when the intake amount is equal to or larger than the target value in step S206 (step S206 - NO), the behavior content determination unit 25 determines whether the intake amount is equal to or smaller than an upper limit value of the target range (step S208). When the intake amount is equal to or smaller than the upper limit value of the target range in step S208 (step S208 - YES), the behavior content determination unit 25 gives a fixed point to the user as a reward for steadily executing the mission (step S209). On the other hand, when the intake amount exceeds the upper limit value of the target range in step S208 (step S208 - NO), the behavior content determination unit 25 notifies the user terminal 3 that the intake amount of the calory and the nutrient is excessively large (an example of an advice) (step S210).

Note that, in the above explanation, whether or not a point is granted is determined according to whether both of the calory and the nutrient is within the target range. However, whether or not a point is granted may be determined separately for the calory and the nutrient.

With the health behavior support device 2 in the second embodiment configured in this way, it is possible to promote health management adapted to characteristics of an individual more. Specifically, a health behavior for health promotion has been proposed to a user. However, in the conventional method, the proposed health behavior does not reflect individual characteristics of the user and is uniformly set. Therefore, it is likely that the proposed health behavior is not always effective for individual users.

In contrast, the health behavior support device 2 in the second embodiment can set, according to a genetic constitution of a user, a health behavior (a mission) recommended for health promotion of the user and quantitatively monitor a behavior state of the user with respect to the set mission. The health behavior support device 2 in the second embodiment can give a reward to the user according to the behavior state of the user with respect to the mission and urge the user to change a behavior. Therefore, the user is capable of efficiently and effectively promoting or maintaining health according to the physical constitution of the user. Since the user is not always aware of the genetic constitution, by executing a mission determined based on genetic characteristics, the user is capable of reducing even a health risk due to the physical constitution that the user is not aware of.

Sources of the reference documents introduced in the second embodiment are described below.

Reference document 7: https://with-aging.com/column/2258/
Reference document 8: https://med.fjtex.co.jp/products/gene/exercise/
Reference document 9: https://www.asken.jp/

Note that, in the first embodiment and the second embodiment, an example is explained in which the health behavior support device 2 is configured as one device. However, the health behavior support device 2 may be implemented using a plurality of information processing devices communicably connected via a network. In this case, the functional units included in the health behavior support device 2 may be implemented to be distributed to the plurality of information processing devices. For example, the database 11, the genetic model generation unit 12, the nongenetic model generation unit 13, the physical constitution estimation unit 14, and the examination item determination unit 15 may be respectively implemented in different information processing devices. For example, the database 21, the mission setting unit 22, the mission management application selection unit 23, the application utilization degree determination unit 24, and the behavior content determination unit 25 may be respectively implemented in different information processing devices. By adopting such a configuration, it is also possible to configure the health behavior support device 2 as a so-called Cloud system.

For example, as an example, a configuration in which a mission setting unit on a Cloud server sets missions for users and mission management applications selected according to the missions are installed in user terminals and perform data coordination with the Cloud server is conceivable. In this case, as an example, a configuration in which the mission management applications acquire information and the like indicating utilization degrees of the applications and evaluation result of behavior content from an application utilization degree determination unit and a behavior content determination unit on the Cloud server and notify the information and the like to the users is conceivable.

The embodiments of the present invention are explained in detail above with reference to the drawings. However, a specific configuration is not limited to the embodiments. Design and the like in a range not departing from the gist of the present invention are also included.

Industrial Applicability

The present invention can provide means for supporting management, support, or promotion of health for organizations such as companies that manage or support health of members, various medical institutions that manage or support health of medical examinees, and individuals who intend to manage and promote health of the individuals.

Reference Signs List

1 Clinical examination item determination device

11 Database
12 Genetic model generation unit
13 Nongenetic model generation unit
14 Physical constitution estimation unit
15 Examination item determination unit
2 Health behavior support device
21 Database
22 Mission setting unit
23 Mission management application selection unit
24 Application utilization degree determination unit
25 Behavior content determination unit
3 User terminal

## Claims

**1.** A clinical examination item determination device that determines a clinical examination item for each user, the clinical examination item determination device comprising:

an individual examination item determination unit that estimates, based on genetic factors of the user and nongenetic factors based on behaviors or habits of the user, a disease that the user develops with high possibility and determines, for the user, as an individual examination item, an examination item for detecting presence or absence of the estimated disease;
a common examination item determination unit that determines an examination item for detecting a disease having a high incidence rate in a first organization to which the user belongs or a disease having a high incidence rate in common in the first organization and a second organization having an attribute similar to an attribute of the first organization as a common examination item of people belonging to the first or second organization; and
an examination item determination unit that determines an examination item of the user based on the individual examination item and the common examination item.

**2.** The clinical examination item determination device according to claim 1, wherein lifestyle habits of the user are included in the nongenetic factors.

**3.** The clinical examination item determination device according to claim 1 or 2, wherein the individual examination item determination unit estimates, based on a weighted sum of an incidence rate of a disease estimated based on the genetic factors of the user and an incidence rate of the disease estimated based on the nongenetic factors of the user, possibility that the user develops the disease.

**4.** The clinical examination item determination device according to any one of claims 1 to 3, wherein the individual examination item determination unit estimates, based on a learned model generated by a method of machine learning, the learned model indicating a relationship between human nongenetic factors and incidence rates of diseases, and the nongenetic factors of the user, an incidence rate of the disease for the user.

**5.** The clinical examination item determination device according to any one of claims 1 to 4, wherein the common examination item determination unit selects the common examination item based on a ratio of an incidence rate of a disease in the first or second organization to a general incidence rate of the disease.

**6.** A health behavior support device comprising:

a recommended behavior setting unit that sets, based on genetic information of a user,
for the user, a health behavior that is a behavior recommended to improve health of the user; and
an application selection unit that selects, according to the health behavior set for the user, an application program executable in electronic equipment used by the user, the application program being an application program for supporting implementation of the health behavior by the user.

**7.** The health behavior support device according to claim 6, further comprising:

an application utilization degree determination unit that determines a utilization degree of the application program by the user; and
a behavior content determination unit that, according to the utilization degree of the application program, notifies an advice concerning the health behavior or gives a reward corresponding to an implemented health behavior to the user.

**8.** A clinical examination item determination method for determining a clinical examination item for each user, the clinical examination item determination method comprising:

an individual examination item determination step for estimating, based on genetic factors of the user and non-genetic factors based on behaviors or habits of the user, a disease that the user develops with high possibility and determining, for the user, as an individual examination item, an examination item for detecting presence or absence of the estimated disease;
an organization examination item determination step for determining an examination item for detecting a disease having a high incidence rate in a first organization to which the user belongs or a disease having a high incidence rate in common in the first organization and a second organization having an attribute similar to an attribute of the first organization as a common examination item of people belonging to the first or second organization; and
an examination item determination step for determining an examination item of the user based on the individual examination item and the common examination item.

**9.** A health behavior support method comprising:

a recommended behavior setting step for setting, based on genetic information of a user, for the user, a health behavior that is a behavior recommended to improve health of the user; and
an application determination step for determining, according to the health behavior set for the user, an application program executable in electronic equipment used by the user, the application program being an application program for supporting implementation of the health behavior by the user.

**10.** A computer program for causing a computer to function as the clinical examination item determination device according to any one of claims 1 to 5.

**11.** A computer program for causing a computer to function as the health behavior support device according to claim 6 or 7.

**Fig. 1**

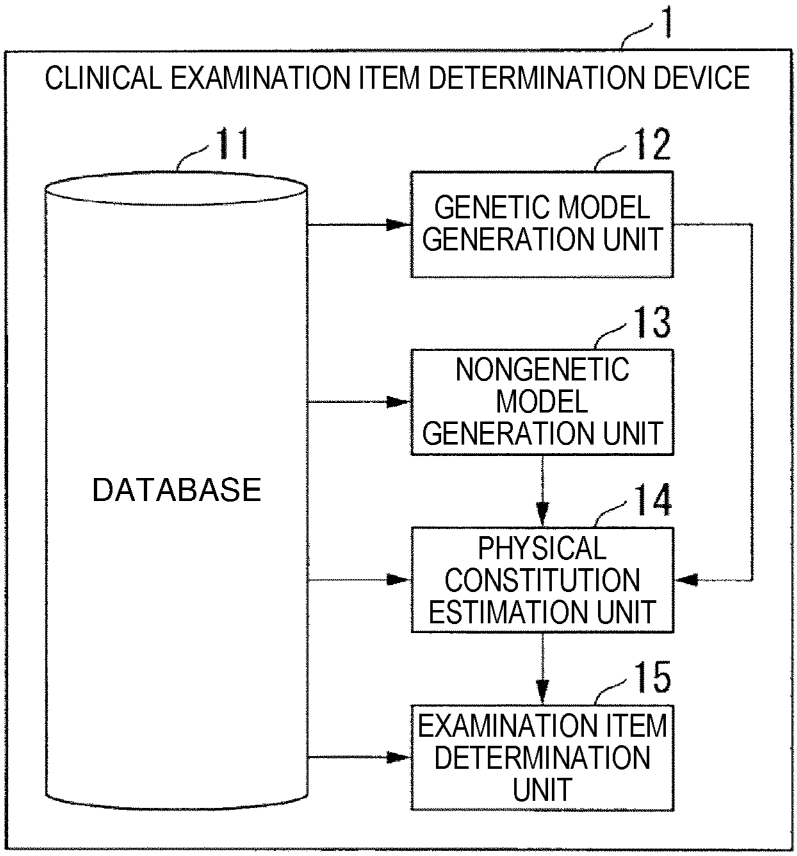
1
CLINICAL EXAMINATION ITEM DETERMINATION DEVICE
11
DATABASE
12
GENETIC MODEL GENERATION UNIT
13
NONGENETIC MODEL GENERATION UNIT
14
PHYSICAL CONSTITUTION ESTIMATION UNIT
15
EXAMINATION ITEM DETERMINATION UNIT

# Fig. 2

| | NN | RN | RR |
|---|---|---|---|
| CONTROL | A | B | C |
| CASE | D | E | F |

# Fig. 3

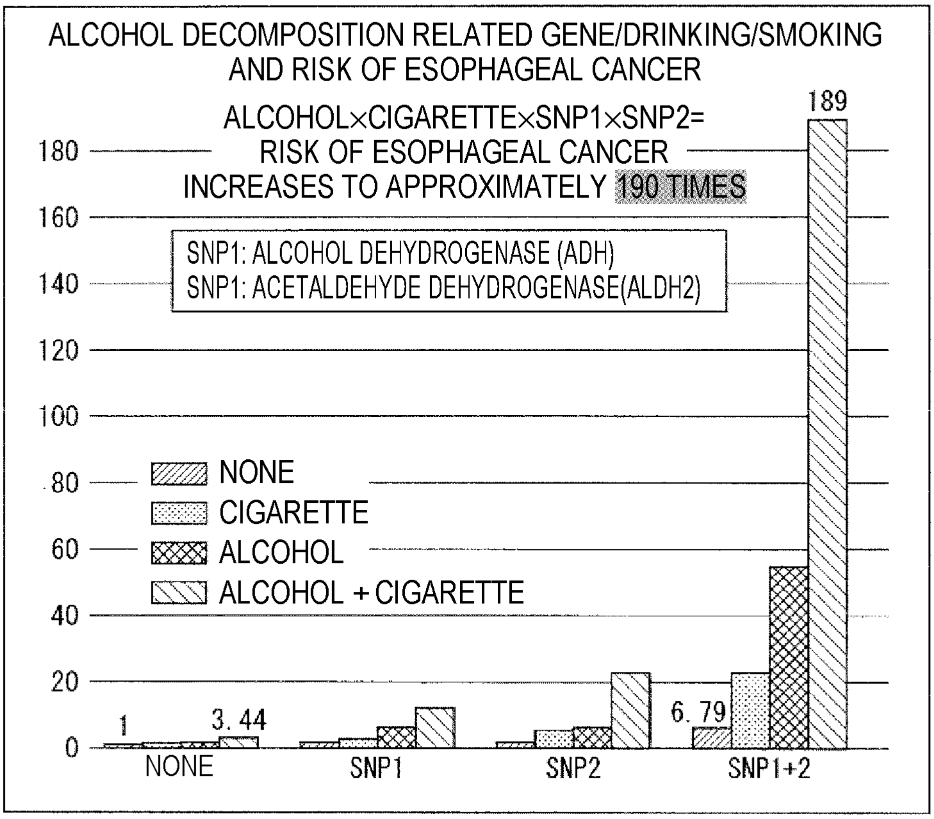
ALCOHOL DECOMPOSITION RELATED GENE/DRINKING/SMOKING AND RISK OF ESOPHAGEAL CANCER
ALCOHOL×CIGARETTE×SNP1×SNP2=
RISK OF ESOPHAGEAL CANCER
INCREASES TO APPROXIMATELY 190 TIMES
SNP1: ALCOHOL DEHYDROGENASE (ADH)
SNP1: ACETALDEHYDE DEHYDROGENASE(ALDH2)
NONE
CIGARETTE
ALCOHOL
ALCOHOL + CIGARETTE
189
1
3. 44
6. 79
0
20
40
60
80
100
120
140
160
180
NONE
SNP1
SNP2
SNP1+2

# Fig. 4

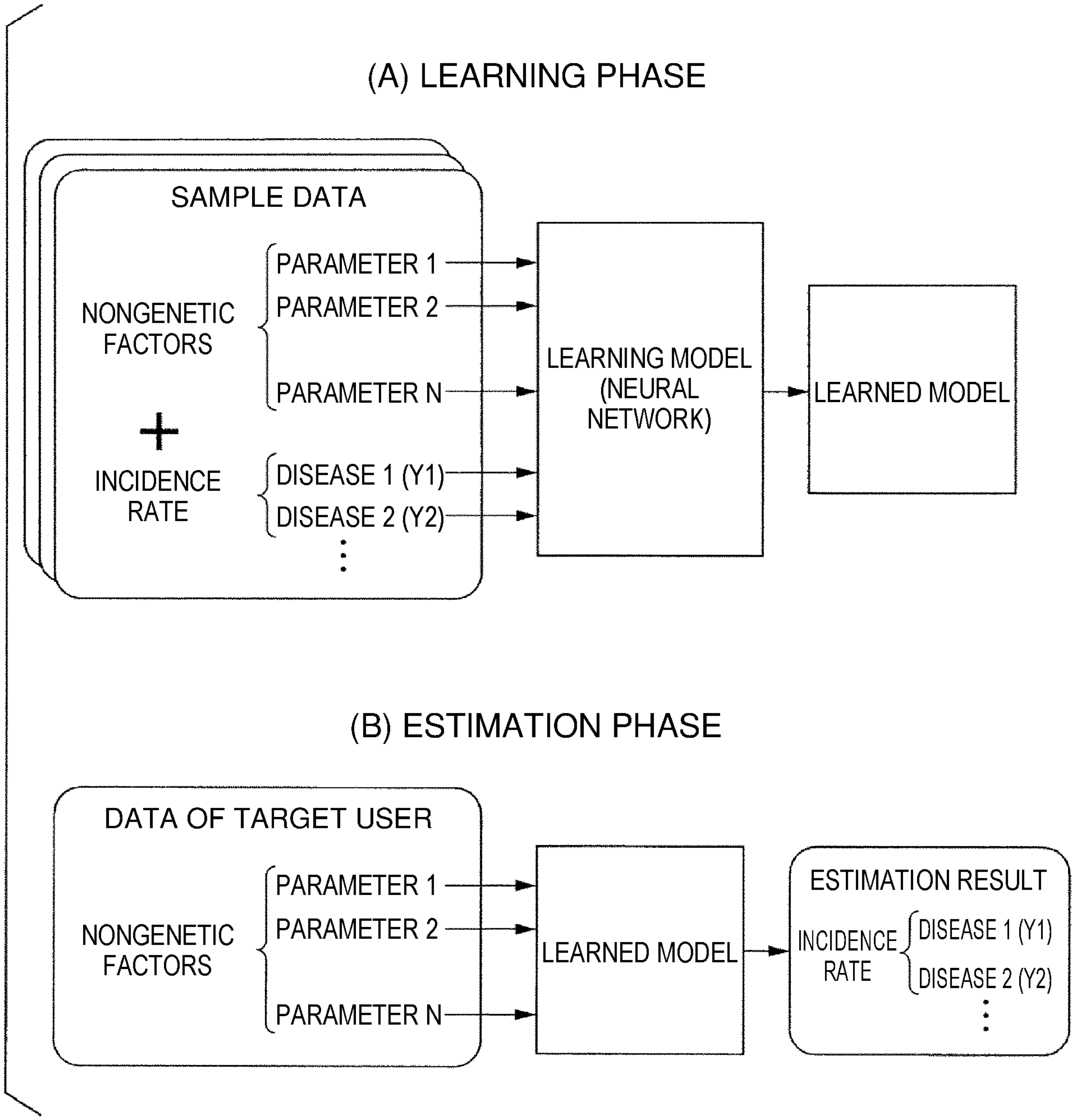
(A) LEARNING PHASE
SAMPLE DATA
NONGENETIC FACTORS
PARAMETER 1
PARAMETER 2
PARAMETER N
+
INCIDENCE RATE
DISEASE 1 (Y1)
DISEASE 2 (Y2)
LEARNING MODEL (NEURAL NETWORK)
LEARNED MODEL
(B) ESTIMATION PHASE
DATA OF TARGET USER
NONGENETIC FACTORS
PARAMETER 1
PARAMETER 2
PARAMETER N
LEARNED MODEL
ESTIMATION RESULT
INCIDENCE RATE
DISEASE 1 (Y1)
DISEASE 2 (Y2)

# Fig. 5

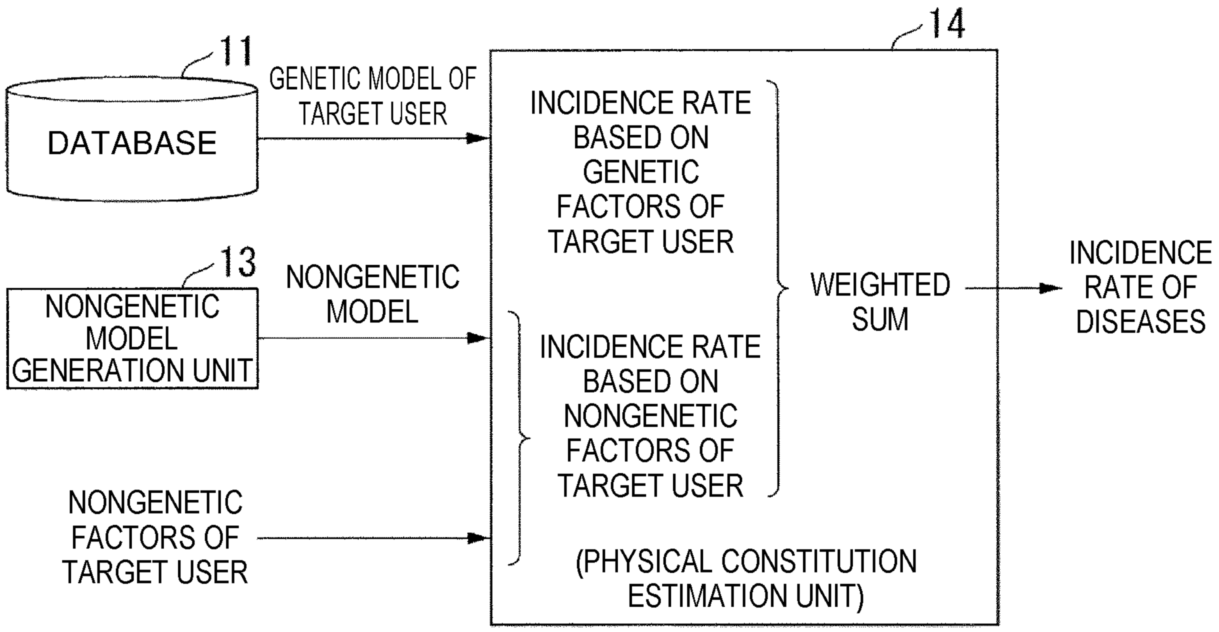
11
DATABASE
GENETIC MODEL OF TARGET USER
13
NONGENETIC MODEL GENERATION UNIT
NONGENETIC MODEL
NONGENETIC FACTORS OF TARGET USER
14
INCIDENCE RATE BASED ON GENETIC FACTORS OF TARGET USER
INCIDENCE RATE BASED ON NONGENETIC FACTORS OF TARGET USER
WEIGHTED SUM
(PHYSICAL CONSTITUTION ESTIMATION UNIT)
INCIDENCE RATE OF DISEASES

## Fig. 6

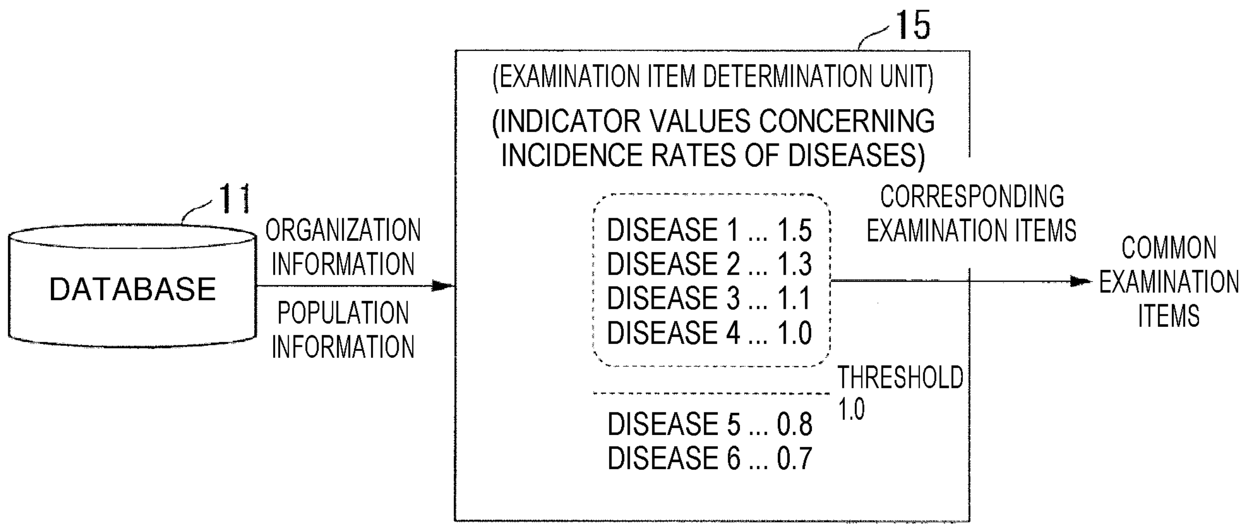
15
(EXAMINATION ITEM DETERMINATION UNIT)
(INDICATOR VALUES CONCERNING INCIDENCE RATES OF DISEASES)
11
DATABASE
ORGANIZATION INFORMATION
POPULATION INFORMATION
DISEASE 1 ... 1.5
DISEASE 2 ... 1.3
DISEASE 3 ... 1.1
DISEASE 4 ... 1.0
THRESHOLD 1.0
DISEASE 5 ... 0.8
DISEASE 6 ... 0.7
CORRESPONDING EXAMINATION ITEMS
COMMON EXAMINATION ITEMS

# Fig. 7

(A)

| | INCIDENCE RATE [%] |
|---|---|
| GINGIVITIS/PERIODONTAL DISEASE | R1 |
| ALLERGIC RHINITIS | R2 |
| ACUTE UPPER RESPIRATORY TRACT INFECTION | R3 |
| REFRACTION/ACCOMMODATION DISORDER | R4 |
| ACUTE BRONCHITIS/BRONCHIOLITIS | R5 |

(B)

| | MEDICAL EXPENSES |
|---|---|
| GINGIVITIS/PERIODONTAL DISEASE | C1 |
| OTHER DIGESTIVE DISEASES | C2 |
| DIABETES | C3 |
| HYPERTENSIVE DISEASE | C4 |
| OTHER MALIGNANT NEOPLASMS (TUMORS) | C5 |

Fig. 8

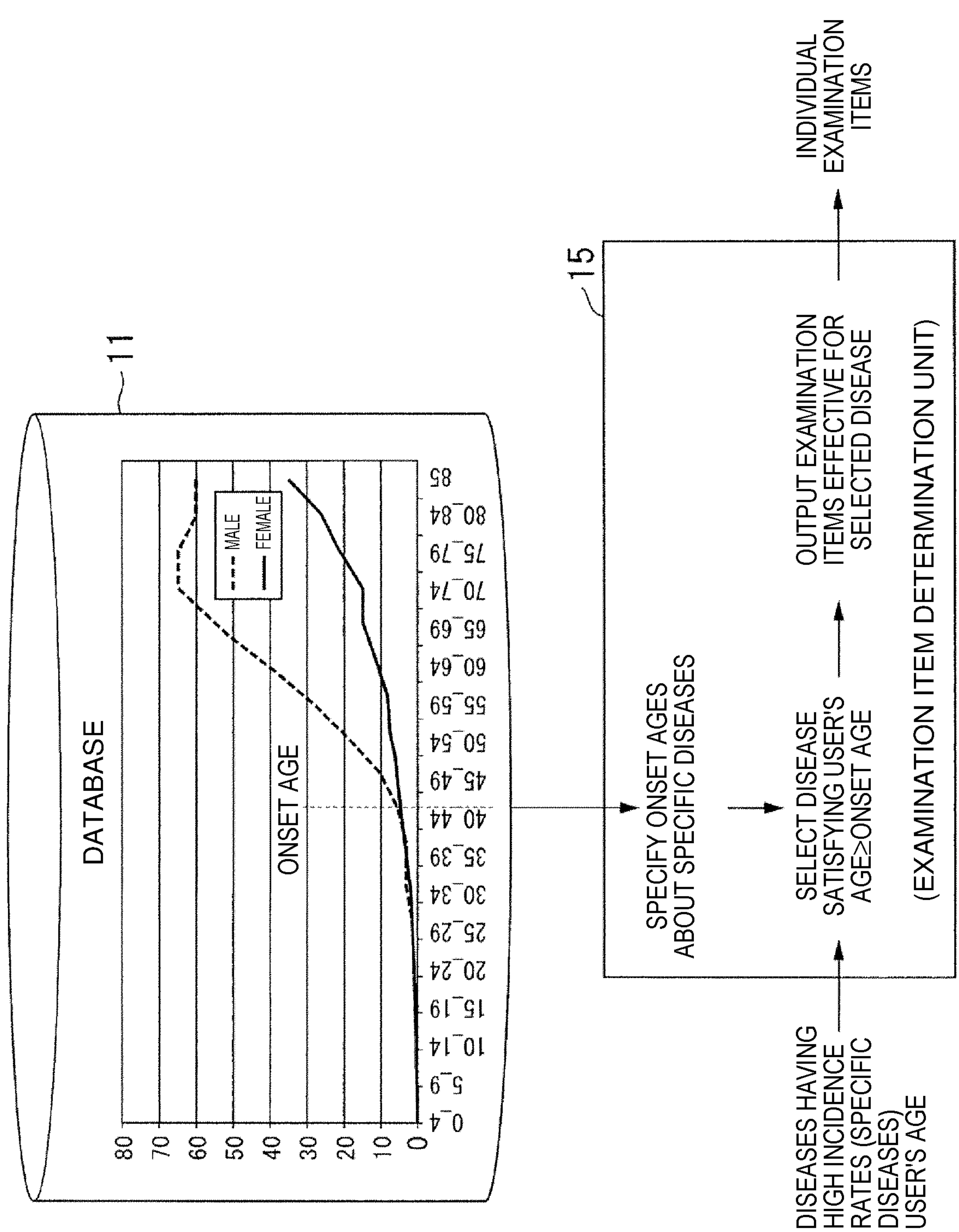
11
DATABASE
ONSET AGE
80
70
60
50
40
30
20
10
0
0_4
5_9
10_14
15_19
20_24
25_29
30_34
35_39
40_44
45_49
50_54
55_59
60_64
65_69
70_74
75_79
80_84
85
MALE
FEMALE
15
SPECIFY ONSET AGES ABOUT SPECIFIC DISEASES
SELECT DISEASE SATISFYING USER'S AGE≥ONSET AGE
OUTPUT EXAMINATION ITEMS EFFECTIVE FOR SELECTED DISEASE
(EXAMINATION ITEM DETERMINATION UNIT)
· DISEASES HAVING HIGH INCIDENCE RATES (SPECIFIC DISEASES)
· USER'S AGE
INDIVIDUAL EXAMINATION ITEMS

Fig. 9

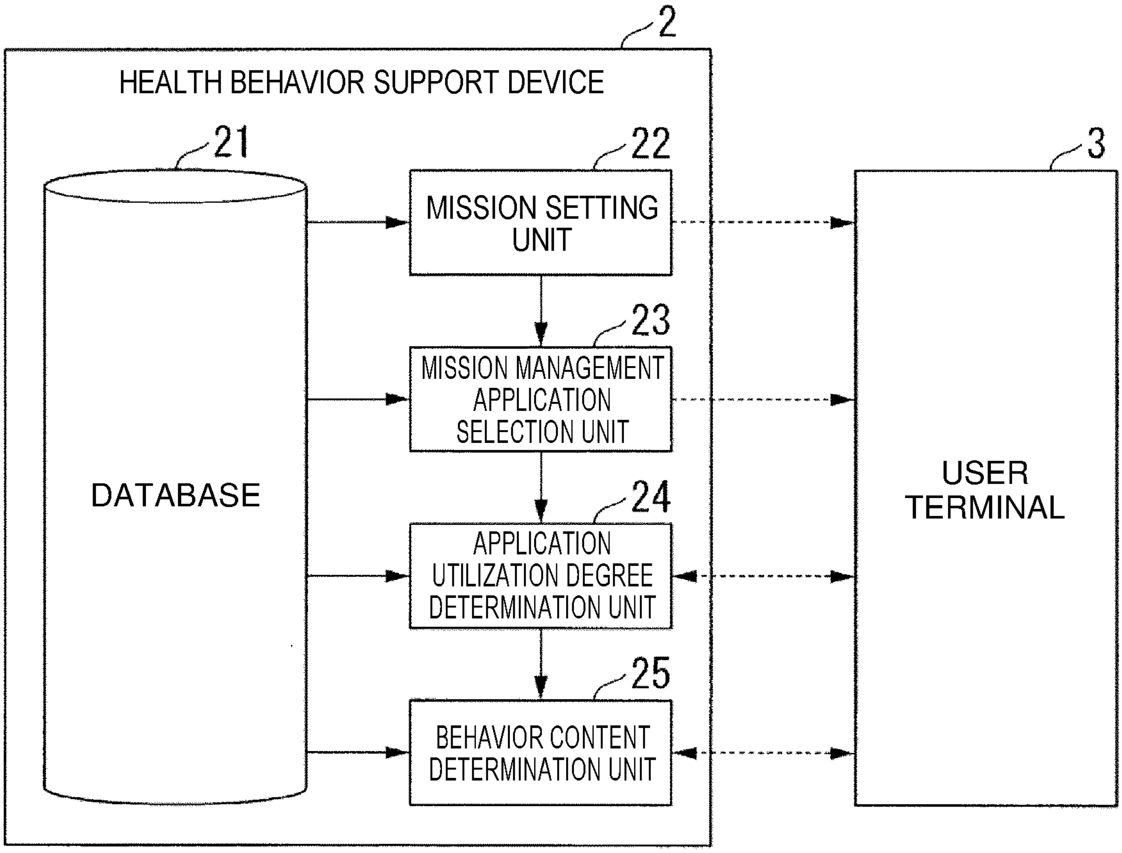
2
HEALTH BEHAVIOR SUPPORT DEVICE
21
DATABASE
22
MISSION SETTING UNIT
23
MISSION MANAGEMENT APPLICATION SELECTION UNIT
24
APPLICATION UTILIZATION DEGREE DETERMINATION UNIT
25
BEHAVIOR CONTENT DETERMINATION UNIT
3
USER TERMINAL

Fig. 10

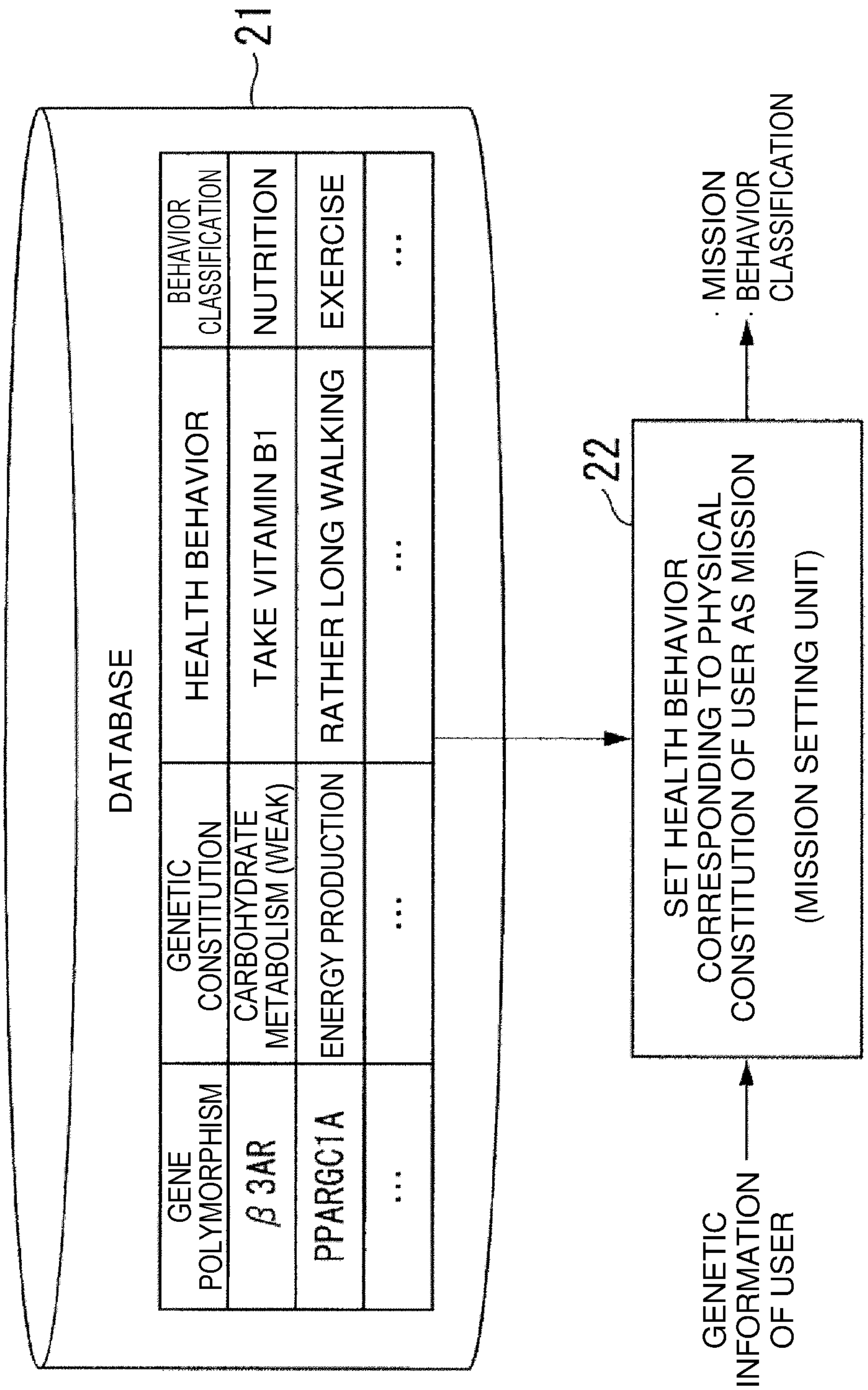
21
DATABASE
GENE POLYMORPHISM
GENETIC CONSTITUTION
HEALTH BEHAVIOR
BEHAVIOR CLASSIFICATION
β 3AR
CARBOHYDRATE METABOLISM (WEAK)
TAKE VITAMIN B1
NUTRITION
PPARGC1A
ENERGY PRODUCTION
RATHER LONG WALKING
EXERCISE
...
22
GENETIC INFORMATION OF USER
SET HEALTH BEHAVIOR CORRESPONDING TO PHYSICAL CONSTITUTION OF USER AS MISSION
(MISSION SETTING UNIT)
· MISSION
· BEHAVIOR CLASSIFICATION

Fig. 11

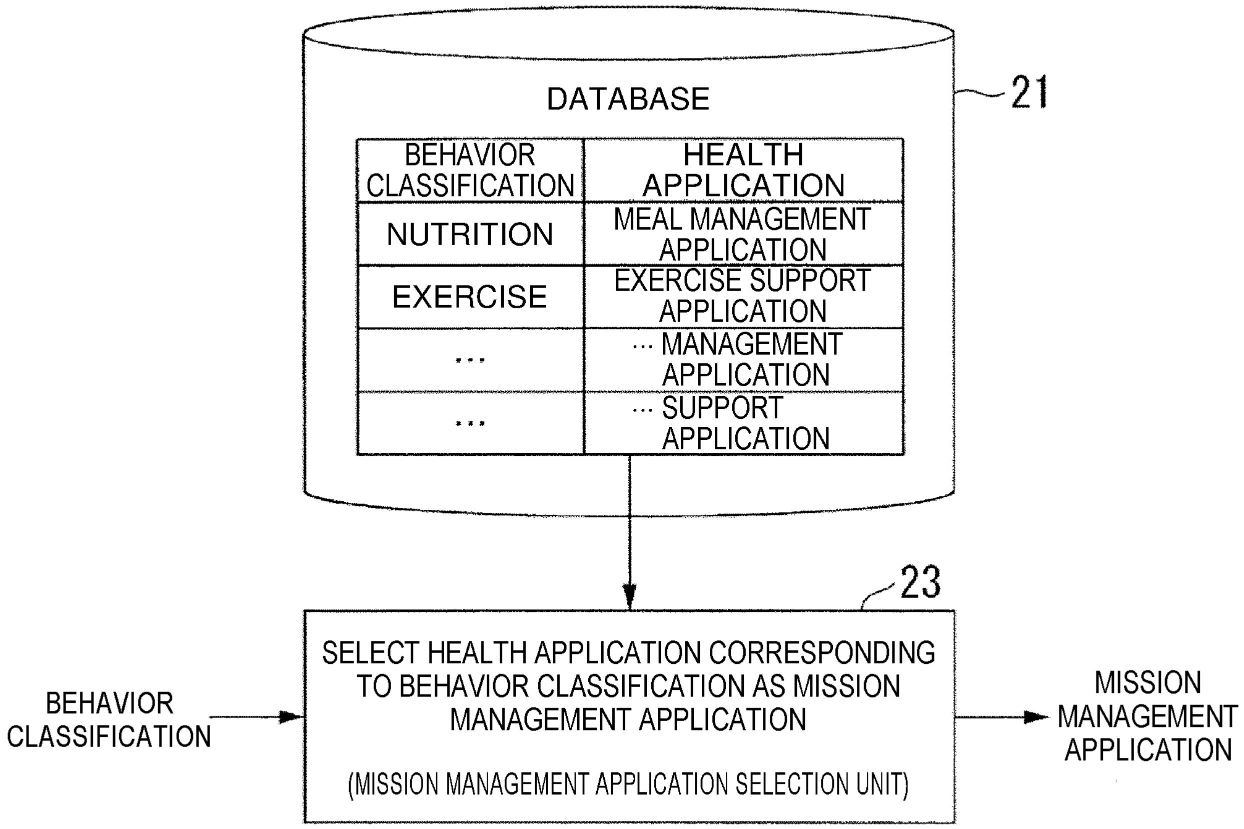
DATABASE
21
BEHAVIOR CLASSIFICATION
HEALTH APPLICATION
NUTRITION
MEAL MANAGEMENT APPLICATION
EXERCISE
EXERCISE SUPPORT APPLICATION
...
... MANAGEMENT APPLICATION
...
... SUPPORT APPLICATION
23
SELECT HEALTH APPLICATION CORRESPONDING TO BEHAVIOR CLASSIFICATION AS MISSION MANAGEMENT APPLICATION
(MISSION MANAGEMENT APPLICATION SELECTION UNIT)
BEHAVIOR CLASSIFICATION
MISSION MANAGEMENT APPLICATION

# Fig. 12

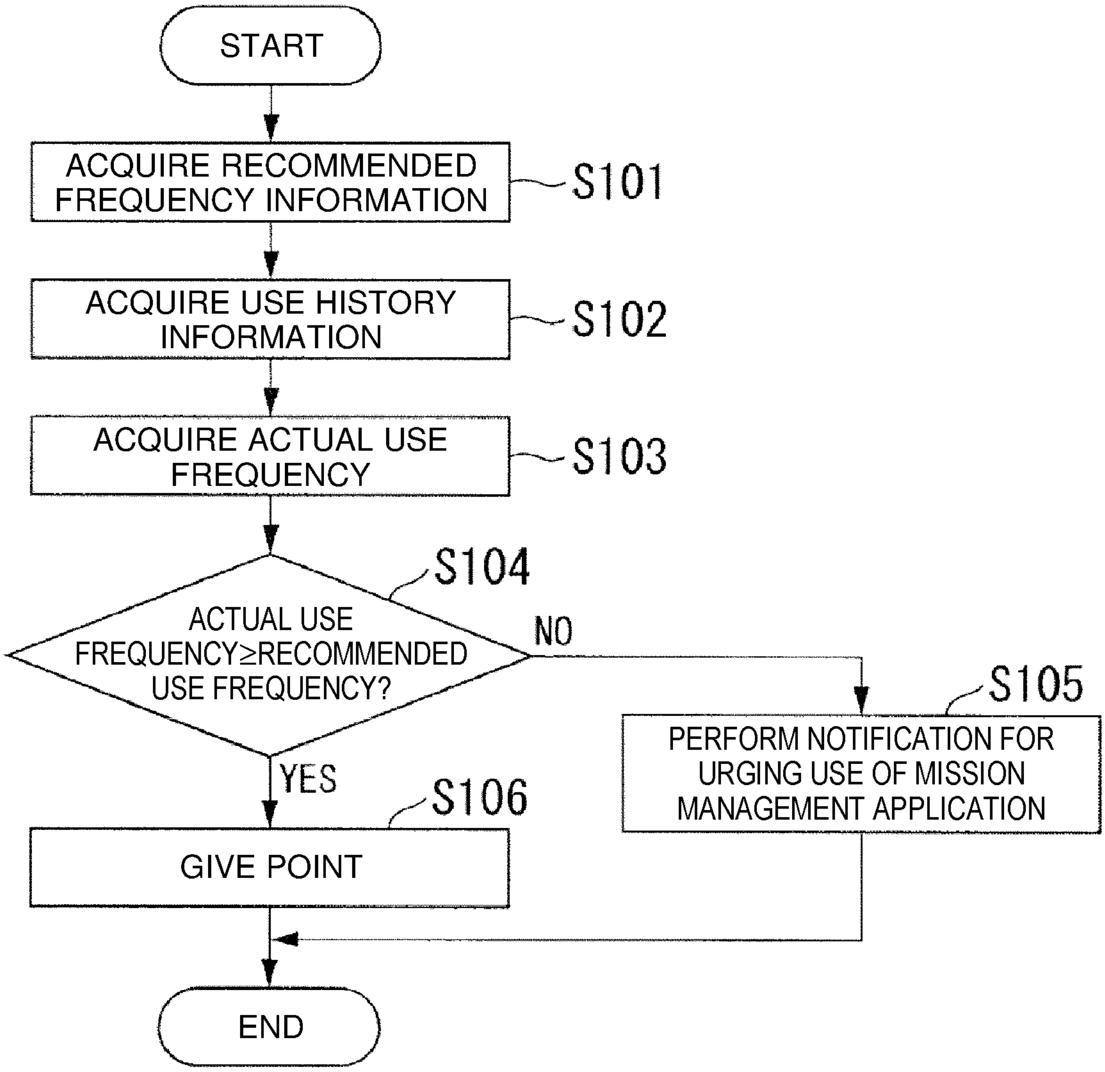
START
ACQUIRE RECOMMENDED FREQUENCY INFORMATION
S101
ACQUIRE USE HISTORY INFORMATION
S102
ACQUIRE ACTUAL USE FREQUENCY
S103
S104
ACTUAL USE FREQUENCY≥RECOMMENDED USE FREQUENCY?
NO
S105
PERFORM NOTIFICATION FOR URGING USE OF MISSION MANAGEMENT APPLICATION
YES
S106
GIVE POINT
END

# Fig. 13

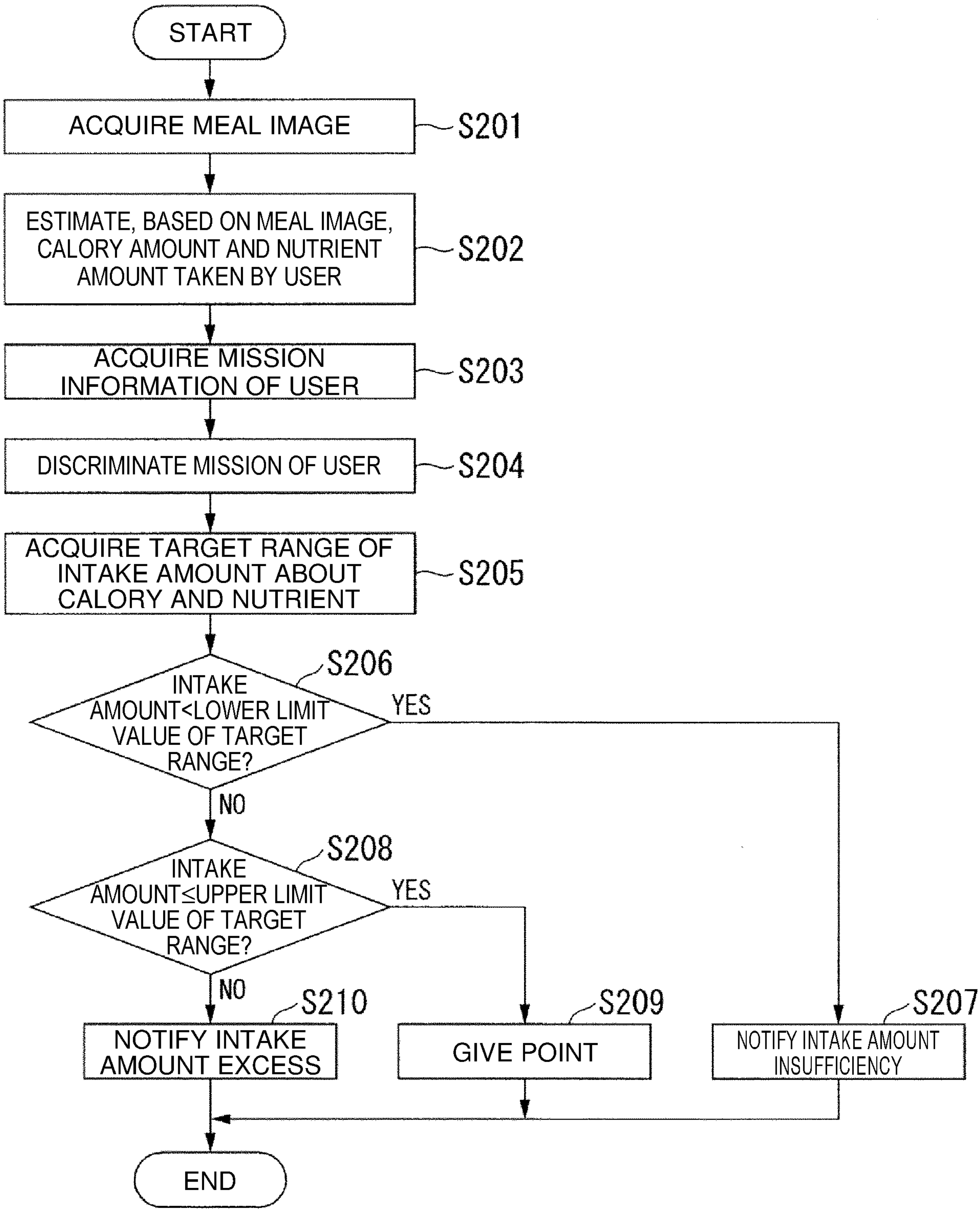
START
ACQUIRE MEAL IMAGE
S201
ESTIMATE, BASED ON MEAL IMAGE, CALORY AMOUNT AND NUTRIENT AMOUNT TAKEN BY USER
S202
ACQUIRE MISSION INFORMATION OF USER
S203
DISCRIMINATE MISSION OF USER
S204
ACQUIRE TARGET RANGE OF INTAKE AMOUNT ABOUT CALORY AND NUTRIENT
S205
S206
INTAKE AMOUNT<LOWER LIMIT VALUE OF TARGET RANGE?
YES
NO
S208
INTAKE AMOUNT≤UPPER LIMIT VALUE OF TARGET RANGE?
YES
NO
S210
NOTIFY INTAKE AMOUNT EXCESS
S209
GIVE POINT
S207
NOTIFY INTAKE AMOUNT INSUFFICIENCY
END

**INTERNATIONAL SEARCH REPORT**

International application No.
PCT/JP2020/014901

A. CLASSIFICATION OF SUBJECT MATTER
Int.Cl. G16H10/40(2018.01)i
FI: G16H10/40

According to International Patent Classification (IPC) or to both national classification and IPC

B. FIELDS SEARCHED

Minimum documentation searched (classification system followed by classification symbols)
Int.Cl. G16H10/40

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | US 2013/0138447 A1 (PATHWAY GENOMICS) 30 May 2013 (2013-05-30), abstract, paragraphs [0022], [0023] | 6-7, 9, 11 |
| Y | JP 2020-27550 A (JAPAN MEDICAL SOLUTIONS INC.) 20 February 2020 (2020-02-20), paragraphs [0004], [0014]-[0016] [0035], [0036], [0038], [0039] | 6-7, 9, 11 |
| A | JP 2008-77603 A (TOSHIBA CORPORATION) 03 April 2008 (2008-04-03), entire text, all drawings | 1-11 |
| A | JP 2013-174951 A (HITACHI MEDICAL CORPORATION) 05 September 2013 (2013-09-05), entire text, all drawings | 1-11 |
| A | JP 2009-273558 A (TOSHIBA CORPORATION) 26 November 2009 (2009-11-26), entire text, all drawings | 1-11 |

☐ Further documents are listed in the continuation of Box C.  ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25 August 2020 | 08 September 2020 |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**
Information on patent family members

International application No.
PCT/JP2020/014901

| | | |
|---|---|---|
| US 2013/0138447 A1 | 30 May 2013 | (Family: none) |
| JP 2020-27550 A | 20 February 2020 | (Family: none) |
| JP 2008-77603 A | 03 April 2008 | US 2008/0076976 A1<br>entire text, all drawings<br>CN 101169863 A |
| JP 2013-174951 A | 05 September 2013 | (Family: none) |
| JP 2009-273558 A | 26 November 2009 | (Family: none) |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *A gene 'ACACB' relating to diabetic nephropathy is found, http://www.riken.jp/pr/press/2010/20100212*
- *olygene risk score PRS: Integrate thousands of gene mutations to predict a disease, https://www.rgare.com/docs/default-source/subsite-materials/japan-reflections/reflections-vol-45.pdf*
- *Quality of scientific grounds, https://mycode.jp/benefits/basis.html*
- *Approach to a relation between liquor and cigarette and esophageal cancer, https://biobankjp.org/cohort_1st/public/tsushin07/biobank _tsushin07_02.html*
- *Influence of inheritance is 87% for mathematics, 66% for IQ, and 59% for income! What are astonishing latest research results?, https://dot.asahi.com/aera/2019072400072.html?page=1*
- *Latest cancer statistics, https://ganjoho.jp/reg_stat/statistics/stat/summary.html*
- Cancer registration/statistics. Cancer Information Services, National Cancer Cente